# EUROPEAN PATENT SPECIFICATION

(11) **EP 1 574 223 B1**
(45) Date of publication and mention of the grant of the patent: **30.12.2009**
(21) Application number: 05003797.7
(22) Date of filing: 22.02.2005
(51) Int. Cl.: A61L 2/07, A61L 2/24, A61B 1/12

(54) **Autoclave sterilization treatment method and autoclave sterilization apparatus using this treatment method**
Behandlungsmethode zur Autoklavsterilisation und Autoklav zur Durchführung dieser Methode
Méthode pour le traitement de stérilisation en autoclave et autoclave utilisant cette méthode

(30) Priority: 12.03.2004 JP 2004071406
(43) Date of publication of application: 14.09.2005
(73) Proprietor: Olympus Corporation, Tokyo 151-0072 (JP)
(72) Inventor: Watanabe, Atsushi, Hino-shi Tokyo (JP); Nishiie, Takehiro, Hachioji Tokyo (JP); Moriyama, Hiroki, Akishima-shi Tokya (JP)
(74) Representative: von Hellfeld, Axel

(56) References cited:
- FR-A- 2 843 028
- US-A- 5 535 141
- US-A- 6 068 815
- US-A1- 2002 001 551
- PATENT ABSTRACTS OF JAPAN vol. 2003, no. 01, 14 January 2003 (2003-01-14) & JP 2002 263172 A (OLYMPUS OPTICAL CO LTD), 17 September 2002 (2002-09-17)

## Description

### BACKGROUND OF THE INVENTION

### 1. Field of the Invention

This invention relates to an autoclave sterilization treatment method and an autoclave sterilization apparatus using this treatment method and, more particularly, to an autoclave sterilization treatment method of performing sterilization treatment on an endoscope with high-temperature and high-pressure steam as well as an autoclave sterilization apparatus using this treatment method.

### 2. Description of the Related Art

In a medical field, an endoscope has recently been widely used which permits an operator to perform observation and examination of a deep area and the like in a body cavity of a patient by inserting its elongate inserting section into the body cavity and the like and also permits the operator to perform observation, therapeutic treatment and the like by using a therapeutic instrument together as occasion demands. When this type of medical endoscope is in use, a condition necessary for safe use of the endoscope is to securely disinfect and sterilize the same.

For this reason, autoclave sterilization treatment is recently becoming widely used in the case of disinfection and sterilization of endoscopes and the like. Autoclave sterilization treatment can execute sterilization treatment without complicated operations and permits a device to be used immediately after the device has been sterilized, and is also advantageous in terms of running cost. Various proposals have been made as to autoclave sterilization treatment, by JP-A-2000-51323, JP-A-2002-330921 and the like.

Representative conditions for this autoclave sterilization treatment are specified in the American Standards ANSI/AAMI ST37-1992 approved by the American National Standards Institute and published by the Association for the Advancement of Medical Instrumentation and the like. As specific conditions, ANSI/AAMI ST37-1992 states that a sterilization process should be performed as a 4-minute treatment at 132 degrees centigrade (°C) in a prevaccum type process in which pressure reduction is carried out before the sterilization process, and that a sterilization process should be performed as a 10-minute treatment at 132 degrees centigrade (°C) in a gravity type process in which pressure reduction is not carried out before the sterilization process.

The sterilization treatment method disclosed in the above-cited JP-A-2000-51323 uses a communication member which is disposed at a predetermined location so as to selectively set the inside and outside spaces of the endoscope into communication with each other. In this method, the communication between the inside and outside spaces of the endoscope is blocked by the communication member in a washing process, whereas in each of a sterilization process and a loading/unloading process, the inside and outside spaces of the endoscope are set into communication with each other by the communication member. These processes are intended to prevent an exterior member of the endoscope from being swollen and broken due to the pressure difference between the inside and the outside of the endoscope which occurs while autoclave sterilization treatment is being performed.

The endoscope apparatus disclosed in the above-cited JP-A-2002-330921 has a display plate on which setting information necessary for autoclave sterilization treatment is described so that reliable sterilization treatment can be completed.

In the case where autoclave sterilization is to be performed on an endoscope, various preparatory processes are necessary before an actual sterilization treatment is executed. In addition, there are various preparatory processes according to different sterilization treatment methods to be executed. For example, if a method of supplying steam into the inside of an endoscope and sterilizing the same is used, a process for opening a vent hole and the like so as to supply steam into the inside of the endoscope becomes necessary. Otherwise, if a method of sterilizing an endoscope without supplying steam into the inside thereof is used, it becomes necessary to perform a process for fitting a waterproofing cap with a check valve for preventing an exterior member of the endoscope from being swollen and broken due to the difference in air pressure between the inside and the outside of the endoscope.

As described above, in order to enable reliable execution of sterilization treatment of endoscopes, there are a large number of particular precautions corresponding to various kinds of treatment methods or the kinds of objects to be sterilized. However, if a user is to grasp and reliably execute all various kinds of precautions that contain different kinds of content for different treatment methods, the user is forced to perform complicated processes while referring to instruction manuals and the like, or is required to be considerably skilled in the art.

European Patent Application Publication EP 1 550 465 A1 from the proprietor of the present invention also concerns a sterilizer apparatus and a sterilizing method for sterilizing an endoscope. EP 1 550 465 A1 was published on 6 July 2005.

The invention has been made in view of the above-mentioned problems. An object of the invention is to provide an autoclave sterilization treatment method which makes it possible to reliably execute various preparatory processes necessary to perform autoclave sterilization on an endoscope, i.e., unique process items associated with the sterilization treatment of the endoscope, and thereby execute reliable sterilization treatment, as well as an autoclave sterilization apparatus using the treatment method.

### BRIEF SUMMARY OF THE INVENTION

The present invention provides an autoclave sterilization treatment method as defined in claim 1. The invention also provides an autoclave sterilization treatment apparatus as defined in claim 9. Preferred features of the invention are recited in the dependent claims.

Accordingly, there is provided an autoclave sterilization treatment method of sterilizing an endoscope with high-temperature and high-pressure steam, which method includes a check step of requesting confirmation of particular precautions to be taken when the endoscope is to be sterilized with high-temperature and high-pressure steam. Since the check step is incorporated, the reliability of autoclave treatment is improved.

Preferably, the check step includes processing, which displays precautions on a display member, and processing, which confirms whether an instruction signal has been generated, which indicates that an operator has operated an operating location corresponding to each of the precautions. The display member is, for example, a touch panel, and a part of the touch panel can be associated with an operating member. In this manner, it is possible to realize a reliable check.

Preferably, an autoclave treatment apparatus is constructed so that autoclave sterilization treatment cannot proceed to the next step if the check step is not completed. For example, if the check step is not completed, a door through which the endoscope is inserted into a chamber in the autoclave apparatus is prevented from being opened.

Preferably, the precautions correspond to the kind of endoscope subjected to autoclave sterilization. Accordingly, a step of informing the autoclave apparatus of the kind of endoscope is inserted before the check step. The method of informing includes inputting from the operating member and the like.

Examples of the precautions are a request for confirmation of whether a steam vent hole of the endoscope is open, a request for confirmation of whether the endoscope is fitted with a waterproofing cap having a check valve, and the like.

Furthermore, an autoclave sterilization treatment method is provided which makes it possible to highly reliably execute various preparatory processes necessary to perform autoclave sterilization on an endoscope, i.e., particular process items associated with sterilization treatment of the endoscope, and thereby highly reliably execute sterilization treatment as well as an autoclave sterilization apparatus using this treatment method.

### BRIEF DESCRIPTION OF THE SEVERAL VIEWS OF THE DRAWINGS

These and other features, aspects, and advantages of the apparatus and methods of the present invention will become better understood with regard to the following description, appended claims, and accompanying drawings where:
Fig. 1 is a general construction view of an endoscope apparatus corresponding to a high-temperature and high-pressure sterilization method (an autoclave sterilization treatment method) according to one embodiment of the invention;
Fig. 2 is a plan view showing the state in which an endoscope which constitutes part of the endoscope apparatus of Fig. 1 is accommodated in a tray;
Fig. 3 is a cross-sectional view taken along line 3-3 of Fig. 2;
Fig. 4 is a partial magnified cross-sectional view showing the construction of an electrical connection section in the endoscope which constitutes part of the endoscope apparatus of Fig. 1;
Fig. 5 is a partial magnified cross-sectional view showing detail A of Figure 4, which constitutes part of the electrical connector section and includes a vent hole and a filter;
Fig. 6 is a channel diagram diagrammatically showing the construction of a plurality of channels which are disposed in the inside of the endoscope which constitutes part of the endoscope apparatus of Fig. 1;
Fig. 7 is an external perspective view of a high-temperature and high-pressure steam sterilization apparatus (autoclave sterilization apparatus for endoscopes) corresponding to the autoclave sterilization treatment method according to the one embodiment of the invention;
Fig. 8 is a block diagram schematically showing the electrical internal construction of the high-temperature and high-pressure steam sterilization apparatus (autoclave sterilization apparatus for endoscopes) shown in Fig. 7;
Fig. 9 is a view showing a display example of precautions (items to be checked) which are displayed on a display device of the high-temperature and high-pressure steam sterilization apparatus of Fig. 7;
Fig. 10 is a flowchart showing an autoclave sterilization treatment method according to the one embodiment of the invention;
Fig. 11 shows a modification of the flowchart of Fig. 10;
Fig. 12 is a view showing a display example of precautions (items to be checked) which are displayed by using a display device of a high-temperature and high-pressure steam sterilization apparatus (autoclave sterilization apparatus); and
Fig. 13 is a view showing another display example of precautions (items to be checked) which are displayed by using a display device of a high-temperature and high-pressure steam sterilization apparatus (autoclave sterilization apparatus).

### DETAILED DESCRIPTION OF THE INVENTION

Preferred embodiments of the invention will be described below with reference to the accompanying drawings.

First, before reference is made to an autoclave sterilization treatment method according to the present embodiment, schematic constructions of an endoscope apparatus 1 and an autoclave sterilization apparatus to both of which the present embodiment is applied will be described below.

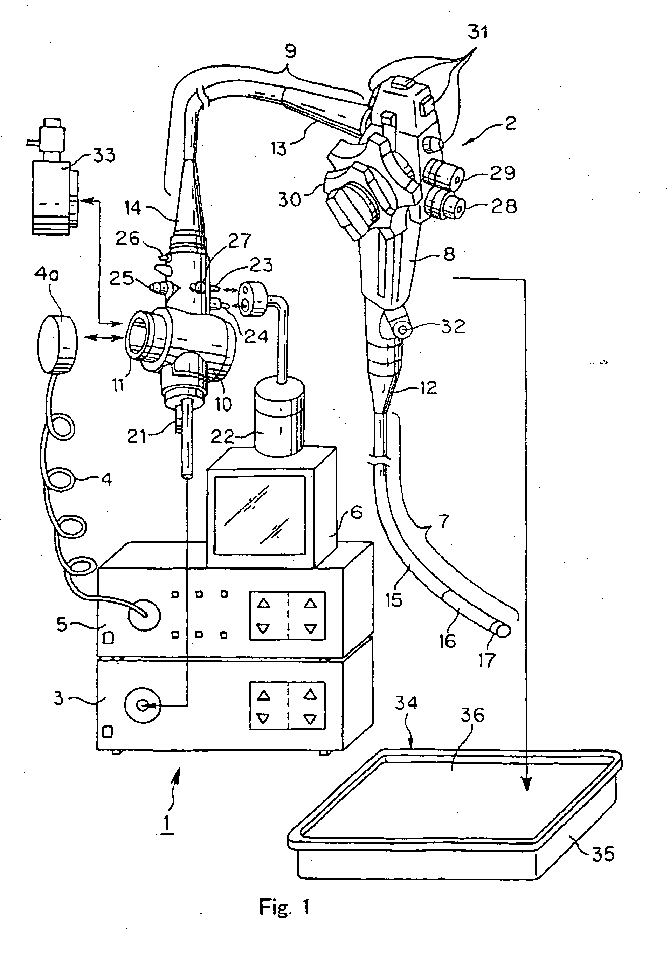

As shown in Fig. 1, an endoscope apparatus 1 to be used for endoscopic examination and the like includes an endoscope 2 provided with image pickup means (not specifically shown), and a light source device 3 removably connected to the endoscope 2 and operative to supply illumination light to a light guide (not specifically shown) provided in the endoscope 2. The endoscope apparatus 1 further includes a video processor 5 which is connected to the endoscope 2 via a signal cable 4 to control the image pickup means of the endoscope 2 and to receive a signal obtained by the image pickup means and perform predetermined signal processing, and a monitor 6 which displays a video image (a subject image) corresponding to a video signal output from the video processor 5, and the like.

After the endoscope 2 has been used for endoscopic examination or the like, the endoscope 2 is washed, and is subsequently subjected to sterilization treatment using high-temperature and high-pressure steam (autoclave sterilization treatment). For this reason, the endoscope 2 is made of members having resistance to high-temperature and high-pressure steam.

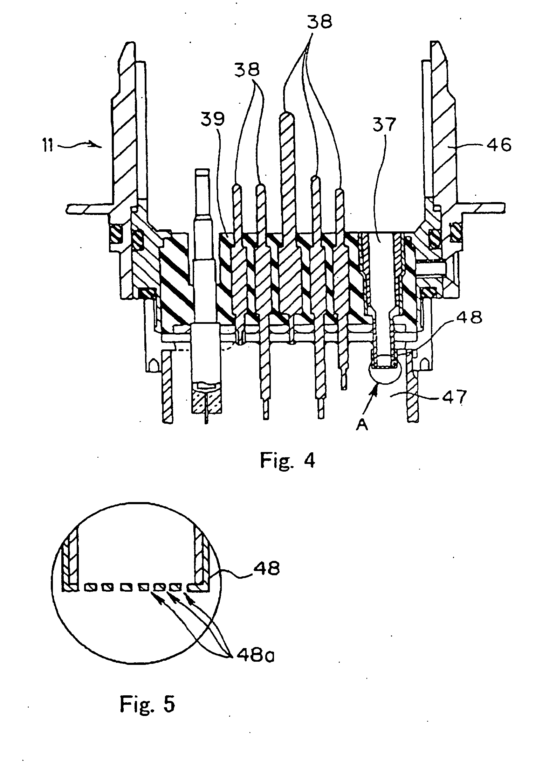

As will be described later, the endoscope 2 has a structure which causes high-temperature and high-pressure steam to positively or forcedly flow into a space 47 (not shown in Fig. 1; refer to Fig. 4) inward of exterior members (an exterior section) such as an inserting section 7 and an operating section 8 and performs sterilization treatment. For this reason, signal wires and the like inward of the endoscope 2 are also made of members having resistance to high-temperature and high-pressure steam.

The endoscope 2 has the elongate inserting section 7 having flexibility, the operating section 8 connected to the proximal side of the inserting section 7, and a connecting cord (a universal cord) 9 extending from one side of the operating section 8 and having flexibility. The endoscope 2 further includes a connector section 10 provided at the distal end of the connecting cord 9 and removably connected to the light source device 3, an electrical connector section 11 provided on one side of the connector section 10, and the like. A connector 4a provided at one end of the signal cable 4 removably connected to the video processor 5 is removably connected to the electrical connector section 11.

The electrical connector section 11 has a vent hole 37 having a predetermined shape to place the inside and the outside of the endoscope 2 into communication with each other (refer to Fig. 4).

An inserting-section supporting member 12 including an elastic member for preventing sharp bending of the inserting section 7 is provided in the connection area between the inserting section 7 and the operating section 8. A similar operating-section supporting member 13 is provided in the connection area between the operating section 8 and the connecting cord 9. In addition, a similar connector-section supporting member 14 is provided in the connection area between the connecting cord 9 and the connector section 10.

The inserting section 7 includes a soft and flexible tube section 15 having flexibility, a bendable section 16 provided at the distal end of the flexible tube section 15 and able to be bent by the operation of the operating section 8, and a tip section 17 disposed at the tip of the inserting section 7 and provided with an observation optical system, an illumination optical system and the like (none of which is shown).

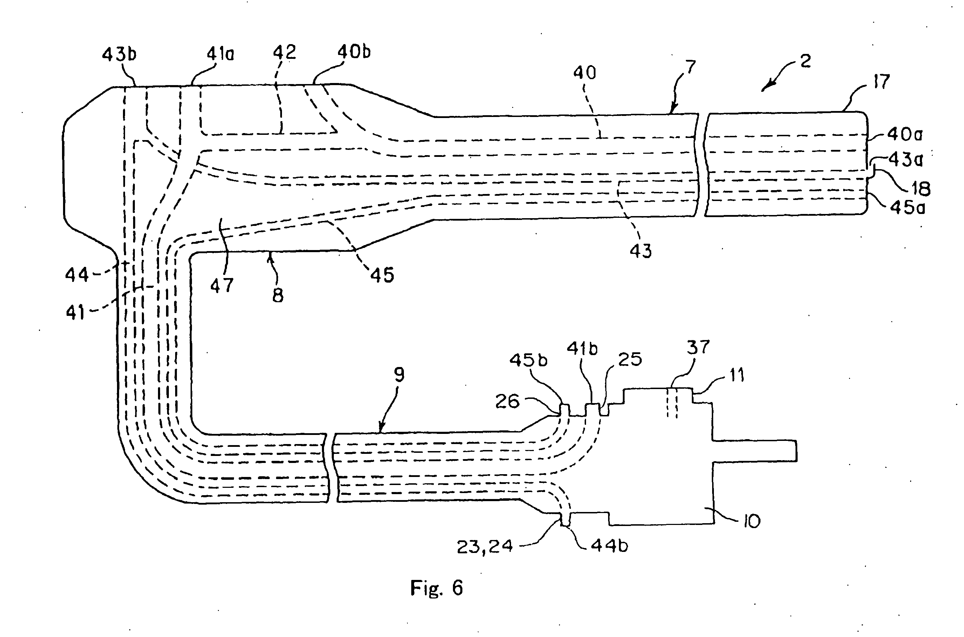

The tip section 17 includes a gas/liquid supply nozzle 18 (not shown in Fig. 1; refer to Fig. 6) for emitting a washing liquid, a gas or the like toward optical members provided on the outside surface of the observation optical system, through a gas supplying operation or a liquid supplying operation. The tip section 17 further includes an aspiration port (not shown) which is an opening formed in the distal end of a therapeutic instrument channel (not shown) through which a therapeutic instrument disposed in the inserting section 7 is to be inserted and which is used for aspirating a liquid from a body cavity, and a liquid emitting port (not shown) which is opened toward an observation object and is used for emitting a liquid toward the same.

The connector section 10 includes a gas-supplying connecting portion 21 removably connected to a gas supply source (not shown) built in the light source device 3, as well as a liquid-tank-pressurizing connecting portion 23 and a liquid-supplying connecting portion 24 both of which are removably connected to a liquid tank 22 which is a liquid supply source. The connector section 10 also includes an aspiration connecting portion 25 connected to an aspiration source (not shown) for performing aspiration. The connector section 10 also includes a liquid-injecting connecting portion 26 connected to liquid supplying means (not shown) for supplying a liquid. The connector section 10 further includes a grounding-terminal connecting section 27 for feeding back to a high-frequency treatment device leak currents which are produced in the endoscope 2 when high-frequency treatment or the like is being performed.

The operating section 8 includes a gas/liquid supplying button 28 for performing a gas supplying operation and a liquid supplying operation, an aspiration operating button 29 for performing an aspiration operation, and a bending operating knob 30 for performing the operation of bending the bendable section 16. The operating section 8 further includes a plurality of remote switches 31 for performing remote control of the video processor 5, a therapeutic instrument inserting port 32 which is an opening communicating with the therapeutic instrument channel, and the like.

A waterproofing cap 33, which is separately provided, can be removably fitted to the electrical connector section 11 of the endoscope 2. This waterproofing cap 33 is constructed including a pressure regulating valve (not shown) in its inside.

When the endoscope 2 is to be washed after the completion of an examination, the waterproofing cap 33 is alternatively fitted in place of the connector 4a, whereby the liquid tightness between the waterproofing cap 33 and the electrical connector section 11 is ensured. Accordingly, a washing liquid is prevented from entering the inside of the electrical connector section 11 from outside, and the liquid is prevented from entering a further inside of the endoscope 2 via the vent hole 37 or a location where contact pins 38 which will be described later are disposed in the inside of the electrical connector section 11.

When sterilization treatment is to be performed, the waterproofing cap 33 is removed. When sterilization treatment is performed during this state, steam is allowed to enter a further inside of the endoscope 2 via the vent hole 37. Namely, at this time, the vent hole 37 serves as a steam inlet.

When high-temperature and high-pressure steam sterilization treatment (hereinafter referred to as autoclave sterilization treatment) is to be performed on the endoscope 2 constructed in the above-mentioned manner, a sterilizing housing case 34 is used as shown in Fig. 1. This sterilizing housing case 34 is made of a tray 35 for housing the endoscope 2 to be sterilized, and a lid member 36 for covering the top opening of the tray 35.

Each of the tray 35 and the lid member 36 has a plurality of vent holes (not shown), and liquid vapor passes through these vent holes during high-temperature and high-pressure steam sterilization treatment.

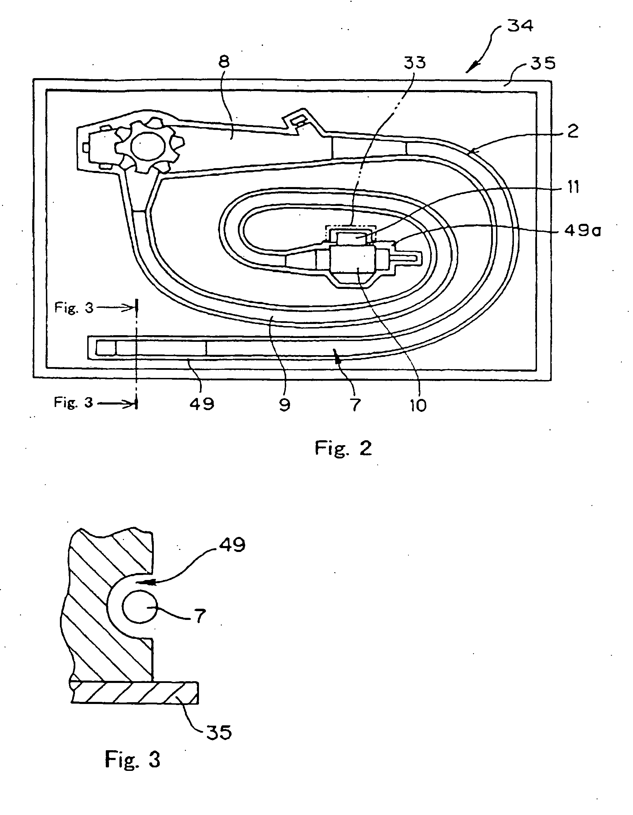

As shown in Fig. 2, the tray 35 includes a restricting section 49 having a concave shape which is formed in a shape corresponding to the shape of the endoscope so that the endoscope 2 can be housed in a predetermined position in the tray 35. Accordingly, the endoscope 2 is housed into the tray 35 in the state shown in Fig. 2.

The restricting section 49 is formed in a concave shape which is slightly larger in size than each section of the endoscope 2 so that the each section can be housed in predetermined positions, respectively. The restricting section 49 has the cross section shown in Fig. 3, for example, in the vicinity of the inserting section 7 of the endoscope 2.

As discussed above, when sterilization treatment is to be performed, the waterproofing cap 33 needs to be removed as mentioned above. For this reason, in a portion 49a in which the electrical connector section 11 of the connector section 10 is to be located when the endoscope 2 is housed in the tray 35, the restricting section 49 is formed in a shape similar to the outline of the connector section 10. Accordingly, even if the connector section 10 is to be housed into the portion denoted by reference numeral 49a with the waterproofing cap 33 fitted as shown by dot-dot-dashed lines in Fig. 2, the connector section 10 cannot be housed in a predetermined position because the outline of the waterproofing cap 33 is larger in size than that of the connector section 10.

As shown in Fig. 4, the electrical connector section 11 has an electrically insulative support plate 39 secured to the inside of a casing 46 to provide a liquid-tight seal. A plurality of contact pins 38 to which are connected the signal wires of the remote switches 31 (refer to Fig. 1) and image pickup signal wires are disposed in this support plate 39.

The support plate 39 of the electrical connector section 11 has the vent hole 37 which places the inside and the outside of the endoscope 2 into communication with each other. Through this vent hole 37, the outside of the endoscope 2 can be made to communicate with the space 47 hermetically sealed in the inside of the endoscope 2 by the exterior section of the endoscope 2.

A filter 48 having a multiplicity of pores 48a which passes steam through but does not allow the passage of matter coarser than steam is disposed as shown in the magnified view of Fig. 5.

In the present embodiment, when the endoscope 2 is sterilized with high-temperature and high-pressure steam, high-temperature and high-pressure steam is made to flow into the space 47 through this vent hole 37, whereby sterilization treatment can be performed in a short time on the outside of a plurality of channels disposed in the inside of the endoscope 2, such as a gas/liquid supplying pipe which communicates with the space 47.

A plurality of channels disposed in the inside of the endoscope 2 will be described below with reference to Fig. 6.

The term "channels" used herein is defined as channels through which a fluid or a therapeutic instrument is passed. A major part of the channels are opened at one end each toward the outside at the tip section 17 of the inserting section 7, and are opened at the other end each toward the outside at locations except the inserting section 7, for example, at the operating section 8 connected to the inserting section 7 and at the connector section 10.

A channel 40 is mainly disposed in the inside of the inserting section 7, and a forward channel end 40a is opened toward the outside at the tip section 17. A rearward channel end 40b is opened toward the outside at a therapeutic-instrument inserting port 32 of the operating section 8. This channel 40 serves as, for example, a channel for insertion of a therapeutic instrument or for aspiration.

A channel 41 is mainly disposed in the inside of the connecting cord 9, and a forward channel end 41a is opened toward the outside at the operating section 8. A rearward channel end 41b is opened toward the outside at the aspiration connecting portion 25 of the connector section 10. This channel 41 serves as, for example, a channel for aspiration.

A channel 42 is mainly disposed in the inside of the operating section 8, and connects the channels 40 and 41. The channel 42 has a forward channel end common to the rearward channel end 40b of the channel 40 and opened toward the outside at the operating section 8. The channel 42 also has a rearward channel end common to the forward channel end 41a of the channel 41 and opened toward the outside at the operating section 8. This channel 42 serves as, for example, a channel for aspiration.

A channel from an aspirator (not shown) is connected to the rearward channel end 41b (the aspiration connecting portion 25) so that aspiration operations can be performed by the aspirator. When the forward channel end 41a and the rearward channel end 40b are closed, aspiration from the forward channel end 40a can be effected through a path extending from the channel 41 to the channel 40 via the channel 42.

A channel 43 is mainly disposed in the inside of the inserting section 7, and a forward channel end 43a is opened toward the outside at the tip section 17. A rearward channel end 43b is opened toward the outside at the operating section 8. This channel 43 serves as a gas/liquid supplying channel for supplying gas and liquid for washing, for example, the lens surface of the tip section 17.

A channel 44 is mainly disposed in the inside of the connecting cord 9, and has a forward channel end common to the rearward channel end 43b of the channel 43 and opened toward the outside at the operating section 8. A rearward channel end 44b is opened toward the outside at the liquid-tank-pressurizing connecting portion 23 and the liquid-supplying connecting portion 24 of the connector section 10.

When gas supply or liquid supply is realized from the rearward channel end 44b (the liquid-tank-pressurizing connecting portion 23 and the liquid-supplying connecting portion 24) with the rearward channel end 43b being closed, gas supply or liquid supply can be provided to the forward channel end 43a.

A channel 45 is mainly disposed in the inside of the inserting section 7 and in the inside of the connecting cord 9, and a forward channel end 45a is opened toward the outside at the tip section 17. A rearward channel end 45b is opened toward the outside at the liquid-injecting connecting portion 26 of the connection section 10. This channel 45 serves as a channel for forward liquid supply for transferring a liquid to, for example, an observation target.

In this manner, a plurality of kinds of channels each having opposite ends opened toward the outside and allowing a fluid and the like to be passed through are disposed in the inside of the endoscope 2. Furthermore, the inserting section 7 and the connecting cord 9 are formed of soft materials, and are formed in not solid but hollow states. In addition, a major part of the channels which are disposed in the inside of the inserting section 7 and in the inside of the connecting cord 9 are respectively arranged in unfixed states in their hollow sections so that the channels can cope with flexible movements of the inserting section 7 and connector cord 9, and although other built-in constituent elements are disposed around these channels, each of the channels is substantially surrounded by a space.

The exterior of the channels (in the interior of the endoscope 2) corresponding to an intermediate section between the opposite ends of the channels communicates with the surrounding space 47. This space 47 communicates with the outside of the endoscope 2 via the vent hole 37 as mentioned previously.

Namely, the exterior of each of the channels can be placed in communication with the outside of the endoscope 2 via the vent hole 37 which is communication means communicating with the space 47. The state of communication between the space 47 and the outside via the vent hole 37 can be changed according to whether the waterproofing cap 33 is fitted or removed.

In the endoscope 2, an intermediate section of a channel which connects one opening and another opening is surrounded by the space 47, and no filler or solid material is provided between the exterior of the channel and the wall of the endoscope. In addition, although various built-in constituent elements and components are present at intermediate locations in a path between the space 47 and the vent hole 37, these built-in constituent elements are arranged so as not to intercept the passage of steam. Accordingly, steam can enter the space 47 via this path without being hindered.

In this manner, in the endoscope 2, the vent hole 37 is formed so that during sterilization treatment, the interior of the endoscope 2 can be made to communicate via the vent hole 37 between the outside of the endoscope 2 and the space 47 which surrounds the plurality of kinds of channels disposed in the interior of the endoscope 2. Accordingly, when prevacuum treatment is performed, the space 47 can also be set to a prevacuum state. In addition, in a subsequent process of performing autoclave sterilization treatment, high-temperature and high-pressure steam can be rapidly supplied not only to the inside spaces of the respective channels but also to the space 47 outside the channels, whereby it is possible to realize a reduction in the time required for the sterilization treatment.

The following description will refer to a high-temperature and high-pressure steam sterilization apparatus (an autoclave sterilization apparatus) which corresponds to a high-temperature and high-pressure sterilization method (hereinafter referred to as an autoclave sterilization treatment method) and performs autoclave sterilization treatment for sterilizing the endoscope 2 housed in the tray 35.

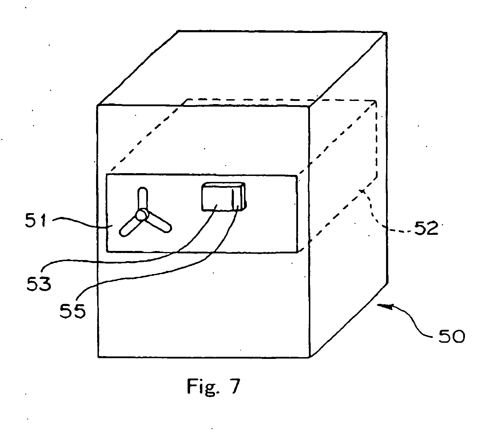

In Fig. 7, an autoclave sterilization apparatus 50 includes a box-shaped body which has a chamber 52 which is made of a housing container disposed in the apparatus and capable of enduring high pressure and high temperature and of serving as a sterilization chamber for high-temperature and high-pressure steam sterilization, and a door 51 disposed to be able to be opened and closed by being rotated toward the front of the chamber 52 as shown in Fig. 7.

Sterilization treatment can be started by opening the door 51, then placing the sterilizing housing case 34 having the endoscope 2 housed in a predetermined position, into the inside of the chamber 52, and subsequently closing the door 51.

The shape of the chamber 52 is selected to assume a housing size barely sufficient to accommodate, for example, only one sterilizing housing case 34 in which the endoscope 2 is housed.

A display device 53 and an operating member 55 are disposed at a predetermined location on the front side of the door 51, i.e., on the side of the door 51 that faces outwardly when the door 51 is closed. The display device 53 displays various kinds of necessary precautions and the like and provides display or the like to request a user to confirm the displayed precautions, when the user is to perform autoclave sterilization treatment with the autoclave sterilization apparatus 50. The operating member 55 is operated by the user when the user is to perform a confirmation operation (a check operation) on each of the precautions displayed by the display device 53.

The display device 53 is made of, for example, a liquid crystal device. The operating member 55 is made of a so-called touch panel or the like which permits the user to turn on and off a switch by touching a particular section on the screen of the display device 53 with his/her finger or the like.

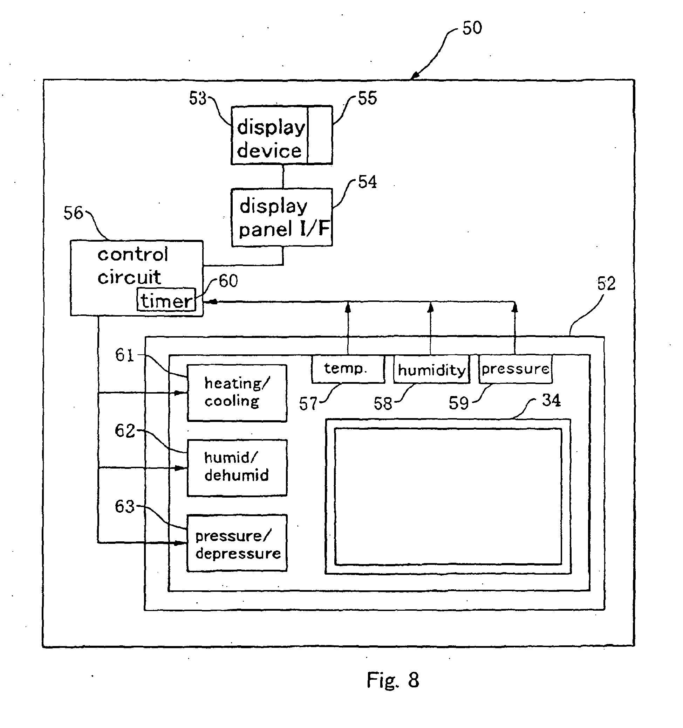

As shown in Fig. 8 in further detail, the autoclave sterilization apparatus 50 includes the above-mentioned chamber 52, a control circuit 56 which performs control and management on temperature, pressure, humidity and the like inside the chamber 52 as well as general electrical control on the autoclave sterilization apparatus 50, a temperature sensor 57 which is disposed in the inside of the chamber 52 and detects temperature inside the chamber 52, a humidity sensor 58 which is similarly disposed in the inside of the chamber 52 and detects humidity inside the chamber 52, and a pressure sensor 59 which is similarly disposed in the inside of the chamber 52 and detects pressure inside the chamber 52. The autoclave sterilization apparatus 50 also includes a heating/cooling unit 61 which communicates with the inside of the chamber 52 and is provided with a heating function and a cooling function for holding the inside of the chamber 52 at a set temperature, a humidification/dehumidification unit 62 which communicates with the inside of the chamber 52 and is provided with a humidification function and a dehumidification function for holding the inside of the chamber 52 at a set humidity, and a pressurization/depressurization unit 63 which communicates with the inside of the chamber 52 and is provided with a pressurization function and a depressurization function for holding the inside of the chamber 52 at a set pressure. Furthermore, the autoclave sterilization apparatus 50 includes a display panel interface 54 which is an interface (I/F) connected between the control circuit 56 and the display device 53 as well as the operating member 55 and operative to transmit display data from the control circuit 56 to the display device 53 and an instruction signal from the operating member 55 to the control circuit 56.

The control circuit 56 has a timer 60 operatively connected thereto for performing time control.

The temperature sensor 57, the humidity sensor 58 and the pressure sensor 59 which are disposed in the inside of the chamber 52 are connected to a port of the control circuit 56, and an output signal from each of the sensors (57, 58 and 59) is inputted to the port of the control circuit 56.

The heating/cooling unit 61, the humidification/dehumidification unit 62 and the pressurization/depressurization unit 63 are connected to a port of the control circuit 56, and each of the units (61, 62 and 63) performs control on temperature, humidity, pressure and the like during autoclave sterilization treatment in accordance with an instruction signal from the control circuit 56.

During autoclave sterilization treatment, information such as the content of sterilization treatment conditions corresponding to the kind of endoscope is displayed in a menu form on the screen of the display device 53. This display is performed under the control of the control circuit 56, and is realized by a signal indicative of the information being transmitted from the control circuit 56 to the display device 53 via the display panel interface 54.

Then, when the kind of endoscope to be subjected to sterilization treatment is selected and inputted by using, for example, the operating member 55, sterilization treatment conditions corresponding to this selection input is set.

In addition, the user can not only set default sterilization treatment conditions corresponding to the kind (type, model, manufacturer, etc.) of endoscope but also arbitrarily change the setting of the sterilization treatment conditions.

When autoclave sterilization treatment is executed, the control circuit 56 starts the timer 60, and performs time control on the operation of the heating/cooling unit 61 and the like according to a period of time which is set under the sterilization treatment conditions being executed.

In addition, in the present embodiment, the control circuit 56 receives output signals from the temperature sensor 57, the humidity sensor 58 and the pressure sensor 59, transfers these respective signals to the heating/cooling unit 61, the humidification/dehumidification unit 62 and the pressurization/depressurization unit 63, and performs feedback control to maintain various settings corresponding to the sterilization treatment conditions being executed.

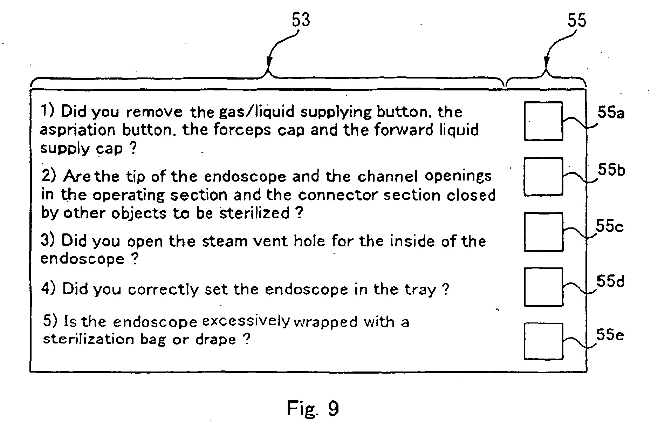

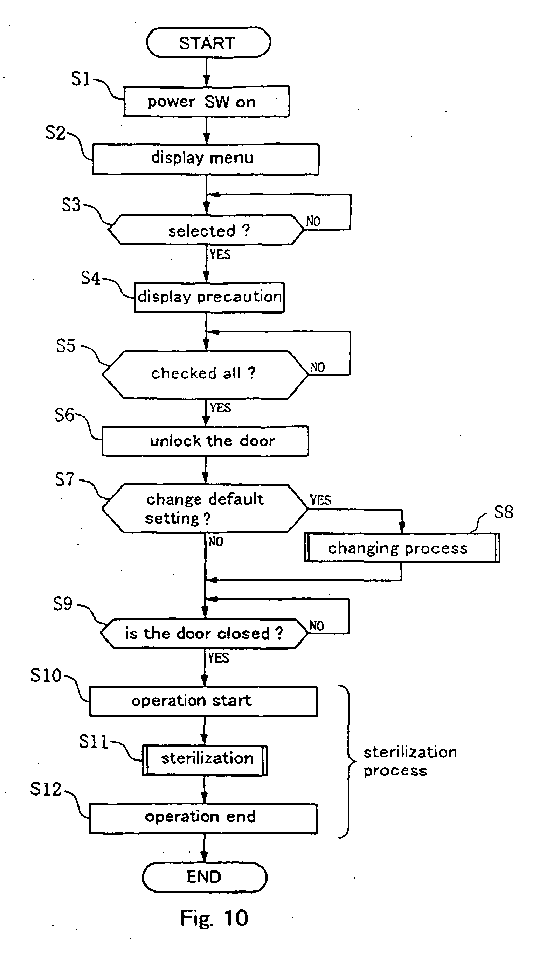

As mentioned above, information such as the content of sterilization treatment conditions corresponding to the kind of endoscope is displayed on the display screen of the display device 53. In a predetermined treatment process (a check process; refer to Fig. 10 which will be mentioned later) immediately before a predetermined sterilization treatment, precautions (items to be checked) corresponding to the set sterilization treatment conditions is displayed. Displayed in this check process are items (1-5) as shown in Fig. 9 by way of example, i.e., precautions corresponding to sterilization conditions which are set when sterilization treatment corresponding to a particular kind of endoscope is to be performed.

The examples shown in Fig. 9 are those of precautions (items to be checked) which are displayed when sterilization treatment is to be performed by supplying steam into the endoscope in the endoscope apparatus mentioned above in the present embodiment, i.e., the inside of the endoscope. In this case, the following precautions and the like are displayed by characters and the like in the form of a list of questions:
1) Did you remove the gas/liquid supplying button, the aspiration button, the forceps cap and the forward liquid supply cap?
2) Are the tip of the endoscope and the channel openings in the operating section and the connector section closed by other objects to be sterilized?
3) Did you open the steam vent hole for the inside of the endoscope?
4) Did you correctly set the endoscope in the tray?
5) Is the endoscope excessively wrapped with a sterilization bag or drape?

Then, when the user performs an operation such as touching with a finger or the like each of locations corresponding to the respective items in an area denoted by reference numeral 55 in Fig. 9 (reference numerals 55a to 55e in Fig. 9), a predetermined check signal corresponding to the checked instruction is produced, and this check signal is transmitted to the control circuit 56 via the display panel interface 54. In this case, the control circuit 56 is not permitted to proceed to the next treatment process, until it receives all check signals.

Representative conditions for high-temperature and high-pressure steam sterilization in which autoclave sterilization apparatuses are generally performed are specified in the American Standards ANSI/AAMI ST37-1992 and the like approved by the American National Standards Institute and published by the Association for the Advancement of Medical Instrumentation. As specific conditions, ANSI/AAMI ST37-1992 states that a sterilization process should be performed as a 4-minute treatment at 132 degrees centigrade (°C) in a prevaccum type in which pressure reduction is carried out before the sterilization process, and that a sterilization process should be performed as a 10-minute treatment at 132 degrees centigrade (°C) in a gravity type in which pressure reduction is not carried out before the sterilization process.

Temperature conditions during sterilization processes in autoclave sterilization treatments differ according to the types of autoclave sterilization apparatus or the periods of time required for the respective sterilization processes, but are in general set within the range of from approximately 115 degrees centigrade (°C) to approximately 138 degrees centigrade (°C). Some types of sterilization apparatus are constructed so that their temperature conditions are variable and can be set to up to approximately 142 degrees centigrade (°C).

Time conditions differ according to the temperature conditions for the respective sterilization processes, but are in general set to approximately 3 to 60 minutes. Some kinds of sterilization apparatus are constructed so that their time conditions are variable and can be set to approximately 100 minutes.

Pressures inside sterilization chambers are in general set to approximately +0.2 MPa with respect to atmospheric pressure.

General prevacuum types of autoclave sterilization processes include a prevacuum process of bringing the inside of a sterilization chamber with contains a subject to be sterilized into a reduced pressure state prior to sterilization and a sterilization process of subsequently supplying high-temperature and high-pressure steam to the sterilization chamber.

The prevacuum process is a process for supplying steam to the details of a device to be sterilized in the subsequent sterilization process, whereby high-temperature and high-pressure steam can be supplied to the entire device to be sterilized, by reducing the pressure inside the sterilization chamber.

The pressure inside the sterilization chamber in the prevacuum process is in general set to approximately -0.07 MPa to - 0.09 MPa with respect to atmospheric pressure.

Some types of autoclave sterilization processes include a drying process for again bringing the inside of the sterilization chamber into a reduced pressure state after the sterilization process for drying the sterilized device. In this process, the inside of the sterilization chamber's pressure is reduced and steam is eliminated from the sterilization chamber, thereby promoting the drying of the device to be sterilized in the sterilization chamber. In this process, the pressure inside the sterilization chamber is in general set to approximately - 0.07 to - 0.09 MPa with respect to atmospheric pressure.

In the present embodiment, as will be described later, autoclave sterilization is performed on the endoscope 2 with the waterproofing cap 33 removed from the electrical connector section 11. Thus, since the vent hole 37 is disposed in the endoscope 2 as mentioned above, sterilization treatment can be completed in a short time.

The size of the vent hole 37 is an important element for the vent hole 37. It is assumed here that the vent hole 37 has a very small (thin) diameter with respect to the volume of the space 47, for example, approximately 0.1 mm. In this case, the speed of pressure variation in the space 47 is slower than that of pressure variation in the chamber 52, so that a time lag occurs until the inside pressures of both are synchronized. Accordingly, from this fact, it is desirable that the size of the vent hole 37 be set to an approximate size with respect to the volume of the space 47.

For example, the size of the vent hole 37 is set to 1 mm or more, more preferably, as large as approximately 5 mm to 10 mm (thick diameter).

In addition, in the case where the area of the vent hole 37 is set to be larger (to a thicker diameter) than the area of a clearance which is the smallest clearance in the space 47 inside the endoscope 2 at a location communicating with the vent hole 37, the vent hole 37 can prevent a bottleneck of steam penetration into the space 47.

The sequence of autoclave sterilization treatment to be executed with the autoclave sterilization apparatus 50 with the endoscope 2 of the endoscope apparatus 1 constructed in the above-mentioned manner being housed in the sterilizing housing case 34 will be described below with reference to Fig. 10.

First, prior to the autoclave sterilization treatment, a target to which this treatment is to be applied, i.e., the endoscope 2, is loaded into the predetermined sterilizing housing case 34.

Then, in step S1, when a user performs an ON operation on a power source switch (not shown) of the autoclave sterilization apparatus 50 to bring the power source switch to an ON state, the autoclave sterilization apparatus 50 goes to a predetermined standby state in the next step S2.

During this standby state, as described previously, information such as the content of sterilization treatment conditions corresponding to the kind of endoscope is displayed in a menu form on the screen of the display device 53. This display is called sterilization condition selection display. While this display is being performed, the autoclave sterilization apparatus 50 waits for the next operation. In addition, a predetermined lock mechanism (not shown) is operating so that the door 51 is prevented from being easily opened.

In step S3, the control circuit 56 checks an instruction signal from the operating member 55 and determines whether the operation of selecting the kind or the like of endoscope to be sterilized, from the sterilization condition selection display. In this step, if it is determined that the operation of selecting sterilization conditions (referred to as the sterilization conditions selection operation) has been performed, sterilization conditions corresponding to that operation are set. Then, the control circuit 56 proceeds to the processing of the next step S4. If the sterilization conditions selection operation has not yet been performed, the control circuit 56 waits for the operation to be performed.

In step S4, the control circuit 56 displays on the display device 53 precautions corresponding to the sterilization conditions which have been set in the above-mentioned step S3. The mode of display of the precautions on the display device 53 is as shown in Fig. 9 by way of example. Then, the control circuit 56 proceeds to the processing of the next step S5. The processing of this step S4 and the processing of the next step S5 constitute the check process.

In step S5, the control circuit 56 determines whether a check operation has been performed on the precautions displayed in the above-mentioned step S4. This confirmation is based on the presence or absence of an instruction signal from the operating member 55. If it is determined in step S5 that check operations have been performed on all the items of the displayed precautions, the control circuit 56 proceeds to the processing of the next step S6.

In step S6, the control circuit 56 controls the lock mechanism of the door 51 and releases the locked state of the door 51. At the same time, the display of the display device 53 is switched to, for example, display for effecting a sterilization-condition-setting changing operation. At this time, since the door 51 is allowed' be opened, the user opens the door 51 and places the sterilizing housing case 34 in which the endoscope 2 is housed in the predetermined position in advance, into a predetermined position inside the chamber 52 of the autoclave sterilization apparatus 50, and then closes the door 51.

Then, in step S7, the control circuit 56 confirms whether the user is to change any setting for default sterilization conditions. This confirmation is performed on the basis of the presence or absence of a signal from the operating member 55 which corresponds to a sterilization-condition-setting changing operation screen displayed on the display device 53. If it is determined in step S7 that a sterilization-condition-setting changing operation is to be performed, the control circuit 56 proceeds to the sterilization-condition-setting changing processing of step S8, and after predetermined change processing has been performed, the control circuit 56 proceeds to the processing of step S9. If a sterilization-condition-setting changing operation is not performed and sterilization treatment based on the default conditions is desired, the control circuit 56 proceeds to the processing of the next step S9.

In step S9, the control circuit 56 confirms whether the door 51 is positively closed. This confirmation is performed on the basis of a closed-state detecting member (not shown) or the like disposed in the door 51. In step S9, the control circuit 56 waits for the door 51 to be positively closed, and proceeds to the processing of the next step S10.

In step S10, the operation of the autoclave sterilization apparatus 50 is started. At this time, the control circuit 56 causes the timer 60 to start a time counting operation, and executes the sterilization treatment of the next step S11. Then, in step S12, when the time counted by the timer 60 exceeds a default time period (time is up), the operation of the autoclave sterilization apparatus 50 is completed (ended). The processing of these steps S10 to S12 constitutes a sterilization process.

As described above, according to the above-mentioned one embodiment, if check operations are not performed on the respective precautions displayed on the display device 53, the locked state of the door 51 is not released, whereby the autoclave sterilization treatment of the endoscope 2 can be constantly reliably executed.

In addition, in the sterilization treatment method according to the above-mentioned one embodiment, if the confirmation operation in the check process of steps S4 to S5 is not performed, the next step, i.e., the release of the locked state of the door 51, is not performed. However, this sequence is not limitative, and the check process has only to be performed before the sterilization process.

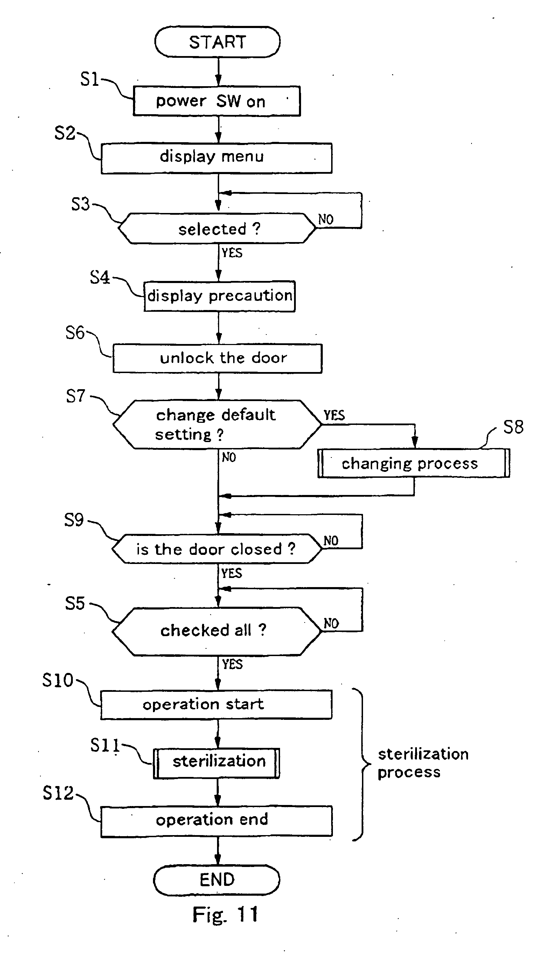

For example, Fig. 11 shows a flowchart in which step S5 is shifted to a position between step S9 and step S10 in the flow of Fig. 10. Even if step S5 is inserted between step S9 and step S10 as shown in Fig. 11, it is possible to obtain advantages similar to those of the above-mentioned first embodiment. Namely, the sterilization treatment method has only to be programmed so that if the confirmation operation in the check process is not performed, the sterilization process is inhibited, i.e., the autoclave sterilization apparatus 50 cannot be operated to perform an actual sterilization treatment.

The operating member 55 of the autoclave sterilization apparatus 50 which is used in the above-mentioned one embodiment is made of a touch panel or the like which is attached to the display device 53. However, the operating member 55 is not limited to this construction, and can also use a general push type of operating switch button or the like.

Furthermore, although the display device 53 is disposed on the front side of the door 51 of the autoclave sterilization apparatus 50, this arrangement is not limitative and the display device 53 may also be disposed at a location other than the door 51.

In addition, the above-mentioned one embodiment has been described with illustrative reference to the case where steam is supplied even into the inside of the endoscope 2 which is an object to be sterilized, and sterilization treatment is also performed on the inside of the endoscope 2. However, the invention is not limited to this example, and can also be easily applied to the case where sterilization treatment is performed without steam being supplied into the inside of an endoscope.

The case where sterilization treatment is performed without steam being supplied into the inside of an endoscope corresponds to the case of an endoscope apparatus of the type disclosed in, for example, JP-A-2002-330921, i.e., the case where when sterilization treatment is to be performed, a waterproofing cap with a check valve is attached to a predetermined position of an endoscope. Exemplary precautions displayed in this case are as follows.
1) Did you remove the gas/liquid supplying button, the aspiration button, the forceps cap and the forward liquid supply cap?
2) Are the tip of the endoscope and the channel openings in the operating section and the connector section closed by other objects to be sterilized?
3) Are you using the waterproofing cap with the check valve? Is the check valve operating correctly?
4) Did you correctly set the endoscope in the tray?
5) Is the endoscope excessively wrapped with a sterilization bag or drape?

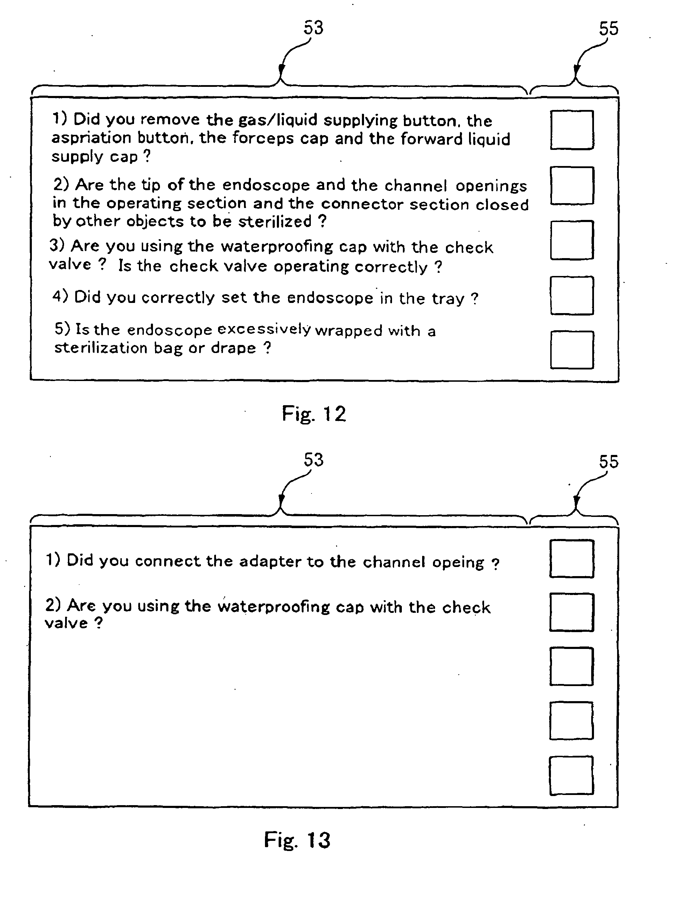

Fig. 12 shows a display example in which the precautions (items to be checked) of the above paragraphs 1) to 5) are displayed by using a display device of a high-temperature and high-pressure steam sterilization apparatus (autoclave sterilization apparatus).

Furthermore, the invention can be easily applied to a case where steam is directly supplied into a channel so that the inside thereof is subjected to sterilization treatment.

An endoscope of the type usable in this case has a construction provided with an adapter member which can directly connect a high-temperature and high-pressure steam sterilization apparatus for use with endoscopes only, which is similar to a washer and sterilizer or the like, for example, to a channel opening formed in an operating section of the endoscope. Exemplary precautions displayed in this case are as follows.
1) Did you connect the adapter to the channel opening?
2) Are you using the waterproofing cap with the check valve?

Fig. 13 shows a display example in which the precautions (items to be checked) of the above paragraphs 1) and 2) are displayed by using a display device of a high-temperature and high-pressure steam sterilization apparatus (autoclave sterilization apparatus).

In this manner, when different sterilization treatments corresponding to different types of endoscopes are to be performed, it is possible to easily cope with such different sterilization treatments by changing content to be displayed on the display screen of the display device 53. In this case, it is possible to obtain advantages similar to those of the above-mentioned one embodiment.

While there has been shown and described what is considered to be preferred embodiments of the invention, it will, of course, be understood that various modifications and changes in form or detail could readily be made. It is therefore intended that the invention be not limited to the exact forms described and illustrated, but should be constructed to cover all modifications that may fall within the scope of the appended claims.

## Claims

1. An autoclave sterilization treatment method for sterilizing an endoscope (2) having at least one channel (40-45) in its inside, with high-temperature and high-pressure steam, the method comprising:
requesting confirmation of one or more particular precautions to be taken prior to sterilization of the endoscope (2) with high-temperature and high-pressure steam; **characterized in that** the requesting of confirmation comprises:
displaying the one or more particular precautions on a display screen of a display device (53) of the autoclave sterilization apparatus; and
confirming an instruction signal generated when an operating member (55) corresponding to each of the one or more particular precautions is operated.

2. An autoclave sterilization treatment method according to claim 1, wherein the method is realized by a program which is programmed so as not to allow the method to proceed from the requesting of confirmation to the sterilization if the conforming is not performed in the requesting of confirmation as to the instruction signal corresponding to each of the one or more particular precautions displayed on the display screen of the display device (53).

3. An autoclave sterilization treatment method according to claim 1, wherein one of the one or more particular precautions in the requesting of confirmation is a precaution which requests confirmation of whether a steam vent hole (37) of the endoscope is opened.

4. An autoclave sterilization treatment method according to claim 1, wherein one of the one or more particular precautions in the requesting of confirmation is a precaution.which requests confirmation of whether a waterproofing cap (33) with a check valve of the endoscope is attached.

5. An autoclave sterilization treatment method according to claim 1, further comprising requesting input of the kind of endoscope (2) to be sterilized prior to the requesting of confirmation, and the one or more particular precautions in the requesting of confirmation are associated with the kind of endoscope input in the requesting of input.

6. An autoclave sterilization treatment method according to claim 1, wherein the confirming comprises displaying the operating location on a part of the display screen of the display device (53).

7. An autoclave sterilization treatment method according to claim 2, wherein the program which does not allow the method to proceed is programmed so as not to allow release of a locked state of a door (51) through which the endoscope is inserted, if the requesting of confirmation is not appropriately completed.

8. An autoclave sterilization treatment method according to claim 2, wherein the program which does not allow the method to proceed is programmed so as not to allow sterilization to be started, if the requesting of confirmation is not appropriately completed.

9. An autoclave sterilization treatment apparatus (50) for sterilizing an endoscope (2) having at least one channel (40-45) in its inside, with high-temperature and high-pressure steam, **characterized in that** the apparatus (50) comprises:
a display device (53) which displays one or more particular precautions to be taken when the endoscope (2) is to be sterilized with high-temperature and high-pressure steam; and
an operating member (55) for performing a confirmation operation on the one or more particular precautions displayed on the display device.

10. An autoclave sterilization treatment apparatus (50) according to claim 9, wherein the operating member (55) comprises a switch for generating an instruction signal in response to the confirmation operation.

11. An autoclave sterilization treatment apparatus (50) according to claim 9, further comprising:
a chamber (52) having a door (51) with a lock, into which the endoscope is to be accommodated; and
a control section (56) which controls the lock, wherein the control section is adapted to not release the lock until the conformation operation with the operating member is completed.

12. An autoclave sterilization treatment apparatus (50) according to claim 9, wherein the display device (53) is a touch panel and the operating member (55) is a part of the touch panel.

13. An autoclave sterilization treatment apparatus (50) according to claim 9, wherein the operating member (55) is a push switch.

14. An autoclave sterilization treatment apparatus (50) according to claim 9, wherein the operating member (55) is also able to input the kind of endoscope (2) to be sterilized, and the display device (53) displays precautions based on the kind of endoscope inputted from the operating member.

## Patentansprüche

1. Behandlungsmethode zur Autoklavsterilisation zum Sterilisieren eines in seinem Inneren wenigstens einen Kanal (40-45) umfassenden Endoskops (2) mit unter hohem Druck stehendem Hochtemperatur-Dampf, wobei das Verfahren umfasst:
Anfordern einer Bestätigung hinsichtlich einer oder mehrerer, vor der Sterilisation des Endoskops (2) mit unter hohem Druck stehendem Hochtemperatur-Dampf zu treffenden speziellen Sicherheitsmaßnahmen;
**dadurch gekennzeichnet, dass** das Anfordern der Bestätigung umfasst:
Anzeigen der einen oder der mehreren speziellen Sicherheitsmaßnahmen auf einem Bildschirm einer Anzeigevorrichtung (53) des Sterilisationsautoklaven; und
Bestätigen eines Befehlssignals, das erzeugt wird, wenn ein jeder der einen oder mehreren speziellen Sicherheitsmaßnahmen entsprechendes Betätigungselement (55) betätigt wird.

2. Behandlungsmethode zur Autoklavsterilisation nach Anspruch 1,
wobei das Verfahren ausgeführt wird durch ein Programm, das so programmiert ist, dass es dem Verfahren nicht erlaubt nach dem Anfordern der Bestätigung die Sterilisation durchzuführen, wenn bei der Bestätigungsanforderung keine Bestätigung hinsichtlich eines Befehlssignals erfolgt, das einer jeden der einen oder mehreren, auf dem Bildschirm der Anzeigevorrichtung (53) angezeigten speziellen Sicherheitsmaßnahmen entspricht.

3. Behandlungsmethode zur Autoklavsterilisation nach Anspruch 1,
wobei eine der einen oder mehreren speziellen Sicherheitsmaßnahmen bei der Bestätigungsanforderung eine Sicherheitsmaßnahme ist, die eine Bestätigung dafür anfordert, dass eine Dampfabzugsöffnung (37) des Endoskops (2) geöffnet wurde.

4. Behandlungsmethode zur Autoklavsterilisation nach Anspruch 1,
wobei eine der einen oder mehreren speziellen Sicherheitsmaßnahmen bei der Bestätigungsanforderung eine Sicherheitsmaßnahme ist, die eine Bestätigung dafür anfordert, dass eine mit einem Kontrollventil versehene Wasserschutzkappe (33) des Endoskops angebracht worden ist.

5. Behandlungsmethode zur Autoklavsterilisation nach Anspruch 1, weiterhin umfassend:
Anfordern der Eingabe der Art des zu sterilisierenden Endoskops (2) vor dem Anfordern einer Bestätigung, wobei die einen oder mehreren speziellen Sicherheitsmaßnahmen bei der Bestätigungsanforderung der Art des bei der Eingabeanforderung eingegebenen Endoskops zugeordnet werden.

6. Behandlungsmethode zur Autoklavsterilisation nach Anspruch 1,
wobei das Bestätigen das Anzeigen der Stelle, an der der Vorgang stattfindet, auf einem Teil des Bildschirms der Anzeigevorrichtung (53) umfasst.

7. Behandlungsmethode zur Autoklavsterilisation nach Anspruch 2,
wobei das Programm, das dem Verfahren nicht erlaubt fortzufahren, so programmiert ist, dass der verriegelte Zustand einer Türe (51), durch die das Endoskop eingebracht wird, nicht aufgehoben werden kann, wenn die Bestätigungsanforderung nicht ordnungsgemäß abgeschlossen worden ist.

8. Behandlungsmethode zur Autoklavsterilisation nach Anspruch 2,
wobei das Programm, das dem Verfahren nicht erlaubt fortzufahren, so programmiert ist, dass es nicht erlaubt die Sterilisation zu beginnen, wenn die Bestätigungsanforderung nicht ordnungsgemäß abgeschlossen worden ist.

9. Autoklav zum Durchführen einer Autoklavsterilisation (50) zum Sterilisieren eines in seinem Inneren wenigstens einen Kanal (40-45) umfassenden Endoskops (2) mit unter hohem Druck stehendem Hochtemperatur-Dampf, **dadurch gekennzeichnet, dass** der Autoklav (50) umfasst:
eine Anzeigevorrichtung (53), die eine oder mehrere spezielle Sicherheitsmaßnahmen anzeigt, die getroffen werden müssen, wenn das Endoskop (2) mit unter hohem Druck stehendem Hochtemperatur-Dampf sterilisiert werden soll; und
ein Betätigungselement (55) zum Ausführen eines Bestätigungsvorgangs hinsichtlich der auf der Anzeigevorrichtung angezeigten einen oder mehreren speziellen Sicherheitsmaßnahmen.

10. Autoklav zum Durchführen einer Autoklavsterilisation (50) nach Anspruch 9,
wobei das Betätigungselement (55) einen Schalter aufweist zum Erzeugen eines Befehlssignals als Reaktion auf den Bestätigungsvorgang.

11. Autoklav zum Durchführen einer Autoklavsterilisation (50) nach Anspruch 9, weiterhin umfassend:
eine Kammer (52) zum Aufnehmen des Endoskops, die eine mit einer Verriegelung versehene Türe (51) aufweist; und
einen die Verriegelung steuernden Steuerbereich (56), wobei der Steuerbereich so ausgebildet ist, dass er die Verriegelung nicht aufhebt bis der Bestätigungsvorgang mit dem Betätigungselement abgeschlossen ist.

12. Autoklav zum Durchführen einer Autoklavsterilisation (50) nach Anspruch 9,
wobei die Anzeigevorrichtung (53) ein Bildschirm-Tastfeld, und das Betätigungselement (55) ein Teil des Bildschirm-Tastfelds ist.

13. Autoklav zum Durchführen einer Autoklavsterilisation (50) nach Anspruch 9,
wobei das Betätigungselement (55) ein Druckschalter ist.

14. Autoklav zum Durchführen einer Autoklavsterilisation (50) nach Anspruch 9,
wobei über das Betätigungselement (55) auch die Art des zu sterilisierenden Endoskops (2) eingegeben werden kann, und die Anzeigevorrichtung (53) Sicherheitsmaßnahmen anzeigt, die auf der Art des von dem Betätigungselement eingegebenen Endoskops basieren.

## Revendications

1. Procédé de traitement de stérilisation par autoclave pour stériliser un endoscope (2) ayant au moins un canal (40-45) en son intérieur, avec un courant à haute température et haute pression, le procédé comprenant :
la demande de confirmation d'une ou plusieurs précaution(s) particulière(s) à prendre avant la stérilisation de l'endoscope (2) avec le courant à haute température et haute pression ;
**caractérisé en ce que** la demande de confirmation comprend :
l'affichage de l'une ou plusieurs précaution(s) particulière(s) sur un écran d'affichage d'un dispositif d'affichage (53) de l'appareil de stérilisation par autoclave ; et
la confirmation d'un signal d'instruction généré lorsqu'un élément opérationnel (55) correspondant à chacune de l'une ou plusieurs précaution(s) particulière(s) est utilisé.

2. Procédé de traitement de stérilisation par autoclave selon la revendication 1, dans lequel le procédé est exécuté par un programme qui est programmé de façon à ne pas autoriser que le procédé continue de la demande de confirmation à la stérilisation si la confirmation n'est pas effectuée dans la demande de confirmation pour ce qui concerne le signal d'instruction correspondant à chacune de l'une ou plusieurs précautions particulières affichées sur l'écran d'affichage du dispositif d'affichage (53).

3. Procédé de traitement de stérilisation par autoclave selon la revendication 1, dans lequel une de l'une ou plusieurs précautions particulières dans la demande de confirmation est une précaution qui demande confirmation qu'un trou de mise à l'air libre de courant (37) de l'endoscope est ouvert.

4. Procédé de traitement de stérilisation par autoclave selon la revendication 1, dans lequel une de l'une ou plusieurs précautions particulières dans la demande de confirmation est une précaution qui demande confirmation qu'un bouchon d'étanchéité à l'eau (33) avec un clapet anti-retour de l'endoscope est fixé.

5. Procédé de traitement de stérilisation par autoclave selon la revendication 1, comprenant en outre la demande d'entrée du type d'endoscope (2) devant être stérilisé avant la demande de confirmation, et l'une ou plusieurs précautions particulières dans la demande de confirmation sont associées avec le type d'endoscope entré lors de la demande d'entrée.

6. Procédé de traitement de stérilisation par autoclave selon la revendication 1, dans lequel la confirmation comprend l'affichage de l'emplacement opérationnel sur une partie de l'écran d'affichage du dispositif d'affichage (53).

7. Procédé de traitement de stérilisation par autoclave selon la revendication 2, dans lequel le programme qui ne permet pas que le procédé continue est programmé de façon à ne pas autoriser la libération d'un état verrouillé d'une porte (51) à travers laquelle l'endoscope est inséré, si la demande de confirmation n'est pas réalisée de façon appropriée.

8. Procédé de traitement de stérilisation par autoclave selon la revendication 2, dans lequel le programme qui ne permet pas que le procédé continue est programmé de façon à ne pas autoriser que la stérilisation commence, si la demande de confirmation n'est pas réalisée de façon appropriée.

9. Appareil de traitement de stérilisation par autoclave (50) pour stériliser un endoscope (2) ayant au moins un canal (40-45) en son intérieur, avec un courant à haute température et haute pression, **caractérisé en ce que** l'appareil (50) comprend :
un dispositif d'affichage (53) qui affiche une ou plusieurs précautions particulières à prendre lorsque l'endoscope (2) doit être stérilisé avec le courant à haute température et haute pression ; et
un élément opérationnel (55) pour exécuter une opération de confirmation sur l'une ou plusieurs précautions particulières affichées sur le dispositif d'affichage.

10. Appareil de traitement de stérilisation par autoclave (50) selon la revendication 9, dans lequel l'élément opérationnel (55) comprend un commutateur pour générer un signal d'instruction en réponse à l'opération de confirmation.

11. Appareil de traitement de stérilisation par autoclave (50) selon la revendication 9, comprenant en outre :
une chambre (52) ayant une porte (51) avec un verrou, dans laquelle l'endoscope doit être reçu ; et
une section de commande (56) qui commande le verrou, dans lequel la section de commande est adaptée à ne pas libérer le verrou avant que l'opération de confirmation avec l'élément opérationnel ne soit effectuée.

12. Appareil de traitement de stérilisation par autoclave (50) selon la revendication 9, dans lequel le dispositif d'affichage (53) est un écran tactile et l'élément opérationnel (55) est une partie de l'écran tactile.

13. Appareil de traitement de stérilisation par autoclave (50) selon la revendication 9, dans lequel l'élément opérationnel (55) est un commutateur poussoir.

14. Appareil de traitement de stérilisation par autoclave (50) selon la revendication 9, dans lequel l'élément opérationnel (55) est également apte à entrer le type d'endoscope (2) devant être stérilisé, et le dispositif d'affichage (53) affiche les précautions sur la base du type d'endoscope entré par l'intermédiaire de l'élément opérationnel.
